## Europäisches Patentamt

## European Patent Office

## Office européen des brevets

(19)

(11) Veröffentlichungsnummer: **0 060 372**
**B1**

(12)

# EUROPÄISCHE PATENTSCHRIFT

(45) Veröffentlichungstag der Patentschrift:
16.01.85

(51) Int. Cl.⁴: **A 61 K 7/50, A 61 K 7/32**

(21) Anmeldenummer: 81890043.3

(22) Anmeldetag: 12.03.81

(54) **Mittel zur Reinigung und Pflege der Haut des Menschen.**

(43) Veröffentlichungstag der Anmeldung:
22.09.82 Patentblatt 82/38

(45) Bekanntmachung des Hinweises auf die Patenterteilung:
16.01.85 Patentblatt 85/3

(84) Benannte Vertragsstaaten:
BE CH DE FR GB IT LI LU NL SE

(56) Entgegenhaltungen:
US - A - 3 962 150

Die Akte enthält technische Angaben, die nach dem
Eingang der Anmeldung eingereicht wurden und die
nicht in dieser Patentschrift enthalten sind.

(73) Patentinhaber: **Gold, Hans F., Dipl.-Ing.,**
**Mauracherstrasse 1, A-5020 Salzburg (AT)**

(72) Erfinder: **Gold, Hans F., Dipl.-Ing., Mauracherstrasse 1,**
**A-5020 Salzburg (AT)**

(74) Vertreter: **Pfeifer, Otto, Dipl.-Ing. et al, Patentanwälte**
**Dipl.-Ing. Dr. techn. Schütz, Alfred Dr. phil. Mrazek,**
**Engelbert Dipl.-Ing. Holzer, Walter Dipl.-Ing. Pfeifer, Otto**
**Fleischmanngasse 9, A-1040 Wien (AT)**

## Beschreibung

Die Erfindung betrifft ein Mittel zur Reinigung und Pflege der Haut des Menschen im Bereich der Ausscheidungsorgane und der angrenzenden Körperteile sowie auch zum Schutz der menschlichen Haut in diesem Bereich, wobei dieses Mittel einen Gehalt an desinfizierend wirkenden hautverträglichen Komponenten und ggf. an zusätzlichen hautverträglichen Komponenten aufweist.

In den AT-PS Nrn. 315381, 326274, 335070 sowie in der DE-PS Nr. 2409570 sind bereits Hautreinigungsmittel und zum Teil auch Hautpflegemittel beschrieben, die zur Anwendung im analen und angrenzenden Bereich, ggf. auch für andere verschmutzte Körperteile, sowie zum Schutz der Haut vor Rötungen und Entzündungen bestimmt sind. Die Anwendung dieser Präparate erfolgt häufig durch Versprühen mittels Treibgasen, und zwar entweder durch direktes Aufsprühen auf die betreffenden Körperpartien, oder indirekt, wobei das jeweilige Mittel vorher auf WC-Papier, Vlies, Watte od. dgl. aufgesprüht wurde. Statt des Sprühens mittels Treibgasen werden auch andere Möglichkeiten des Aufbringens genannt, so etwa mittels Pumpflaschen, Feuchttüchlein od. dgl. Ihrer Zusammensetzung nach sind die aus den AT-PS Nrn. 315381 und 326274 bekannten Produkte im allgemeinen auf Mineralölen, tierischen oder pflanzlichen Ölen und Fetten als Hauptbestandteil, sowie seifenartigen Emulgatoren, auch in Verbindung mit Alkoholen oder Estern, sowie wasserabsorbierenden, desinfizierenden und duftvermittelnden Substanzen aufgebaut. Irgendwelche Rezepturen, die auf die speziellen Erfordernisse weiblicher, männlicher oder der Haut von Säuglingen eingehen, sind in diesen Patentschriften nicht zu finden.

Bei den Hautreinigungspräparaten gemäss der DE-PS Nr. 2409570 und der AT-PS Nr. 335070, die insbesondere zur Verwendung als Schaumspray bestimmt sind, bilden Wollwachsalkohole, gelöst in Mineralölen, zusammen mit Lanolinsäureisopropylestern, die Hauptkomponenten einer Grundzusammensetzung, die ausserdem bestimmte Tenside, nämlich eine Kombination von N,N-Bis-(2-hydroxyäthyl)ölsäureamid und äthoxyliertem Monoäthanolamin, sowie mehr als zur Hälfte Wasser enthält. Auch hier ist eine Anpassung des Präparates, um die Erfordernisse der Haut in jedem Einzelfall erfüllen zu können, nicht möglich, zumal auch die Grundzusammensetzung in engem Rahmen festgelegt ist.

In der US-A Nr. 3962150 ist ein wässeriges, schaumbildendes, homogenes Hautreinigungsmittel beschrieben, das auch für die Körperpflege insbesondere in der Vaginal- und Analregion bestimmt ist und aus 1 bis 15% einer Kombination von nichtionischen und von anionischen Netzmitteln, 1 bis 15% eines alkoholischen Lösungsmittels, umfassend ein- oder mehrwertige Alkohole und Glykoläther, und 70 bis 98% Wasser besteht. Das Mittel kann ggf. noch Duftstoffe und Desinfektionsmittel sowie Fungizide enthalten; es weist aber keinerlei Komponente auf Fett-, Öl- oder Wollwachsbasis auf. Zur Erzielung des zwingend vorgeschriebenen Schaumbildungsvermögens müssen bestimmte Grenzen bezüglich Viskosität und Oberflächenspannung eingehalten werden. Der aus diesem Mittel erzeugte Schaum muss druckempfindlich sein und schon bei leichter Druckanwendung zusammenfallen, wobei auf der mit dem Schaum behandelten Haut kein oder nur ein geringfügiger Film zurückbleiben darf.

Die Erfindung zielt darauf ab, die angeführten Nachteile zu vermeiden und ein neues Hautreinigungs- und -pflegemittel zu schaffen, das schon aufgrund seiner Zusammensetzung eine grössere Variationsbreite ermöglicht. Der Erfindung liegt der Gedanke zugrunde, von den bisher vorgeschlagenen Hauptkomponenten auf Basis von tierischen bzw. pflanzlichen Ölen und Fetten bzw. Wollwachsderivaten aber auch von gänzlich ölfreien Präparaten abzugehen und statt dessen gewisse synthetische waschaktive Stoffe mit leicht steuerbaren und in gewünschter Weise variationsfähigen Eigenschaften in Kombination mit bestimmten hautverträglichen Mineralölen einzusetzen.

Zu diesem Zwecke wird daher ein Mittel zur Reinigung und Pflege der Haut des Menschen im Bereich der Ausscheidungsorgane und der angrenzenden Körperteile, mit einem Gehalt an desinfizierend wirkenden, ggf. noch zusätzlich desodorisierend, erfrischend und/oder parfümierend wirkenden, hautverträglichen Komponenten vorgeschlagen, welches gemäss der Erfindung gekennzeichnet ist durch die folgende Zusammensetzung dieser ggf. versprühbaren Emulsion:

1) synthetische Wasch- und Reinigungsrohstoffe auf Basis von Alkyläthersulfaten bzw. Alkylsulfaten und/oder Fettsäuresarkosiden bzw. Fettsäuremethyltauriden
     1 bis 21, vorzugsweise 4 bis 16 Gew.-%

2) Emulgatoren bzw. selbstemulgierende Grundlagen auf Basis von Alkylpolyglykolätherphosphaten und/oder Fettalkoholpolyglykoläthern, wobei ggf. die selbstemulgierenden Grundlagen an die Stelle der Komponente 1 treten können
     1 bis 12, vorzugsweise 4 bis 8 Gew.-%

3) ggf. Perl- und/oder Seidenglanzgrundstoffe auf Basis von Alkyläthersulfaten
     1 bis 10, vorzugsweise 2 bis 8 Gew.-%

4) viskose hautverträgliche Mineralöle, z.B. Paraffinöl
     2 bis 20, vorzugsweise 4 bis 16 Gew.-%

5) Glycerin und/oder Glykole
     2 bis 15, vorzugsweise 4 bis 10 Gew.-%

6) niedere aliphatische Alkohole
     2 bis 20, vorzugsweise 5 bis 20 Gew.-%

7) Desinfektionsmittelkomponente auf Aldehydbasis      0,1 bis 2 Gew.-%
oder auf Chlorhexidinbasis
     0,01 bis 0,1 Gew.-%

8) Duftstoffkomponente      0,05 bis 1 Gew.-%

9) Wasser      Rest

Das neue Mittel findet insbesondere Anwendung nach der natürlichen Verrichtung, und zwar sowohl für Erwachsene, Kinder und Säuglinge, als auch für Patienten und Inkontinente. Die letztgenannten beschmutzen oft auch andere Hautpartien, wo durch längeres Liegen meist Verkrustungen auftreten. Die erfindungsgemässen Mittel sind auch zur Entfernung derartiger Verkrustungen sowie gegen Verunreinigungen durch Blut und Eiter geeignet. Bei den an die Ausscheidungsorgane angrenzenden Körperteilen, die bei anhaltendem Reiben von Hautpartien gegeneinander, beispielsweise bei langen Fussmärschen, beim Radfahren, Reiten od. dgl. an den Innenseiten der Oberschenkel, betroffen sein können, kann das erfindungsgemässe Mittel ebenfalls erfolgreich angewendet werden.

Die erfindungsgemässen Mittel unterscheiden sich von den bekannten ölhaltigen Präparaten durch die wesentlich andersartige Zusammensetzung der Hauptbestandteile, die von Ölen und Fetten tierischer oder pflanzlicher Herkunft, einschliesslich von Lanolinderivaten, also von Rohstoffen wechselnder Zusammensetzung, frei sind.

Die synthetischen Wasch- und Reinigungsrohstoffe sind für die verschiedensten Hauttypen von Männern, Frauen und Kindern gut geeignet und ergeben in Kombination mit dem Emulgatoren besser in ihren Eigenschaften abstufbare Präparate als jene auf Öl- und Fettbasis. Gleichartige Vorteile werden erzielt, wenn anstelle der genannten Kombination der Komponenten 1 und 2 eine selbstemulgierende Grundlage allein eingesetzt wird. Durch den Einsatz der nichtionogenen Emulgatoren und/oder selbstemulgierenden Grundlagen wird ausserdem die Überführung sämtlicher Komponenten des Präparates, einschliesslich des Paraffinöles, in eine stabile Emulsion sehr erleichtert, was nur einen einfachen Mischvorgang, häufig ohne Erwärmen, notwendig macht.

Entsprechend einer bevorzugten Ausführungsform weist das erfindungsgemässe Mittel einen Gehalt an solchen speziellen Emulgatoren bzw. selbstemulgierenden Grundlagen auf, die als für sich mit Wasser nicht mischbare Komponenten auch die Emulgierung der übrigen Bestandteile des Präparates ermöglichen, was nun eine Zubereitung desselben bei Zimmertemperatur ohne Erwärmung und nachfolgendes Abkühlen erlaubt und somit eine wesentliche Vereinfachung und Verbilligung der Produktion nach sich zieht.

Zusätzlich können als Komponente 3 solche Wirkstoffe vorgesehen sein, durch die auf der Haut ein länger anhaltender Perl- und/oder Seidenglanzeffekt erzielt wird, so dass diese ein besonders appetitliches und attraktives Aussehen gewinnt. Durch die Vielseitigkeit der erfindungsgemäss vorgesehenen Komponenten, wozu das viskose Mineralöl, insbesondere das Paraffinöl, von guter Hautverträglichkeit und festgelegter Qualitätsgarantie zählt, ist es möglich, diese Bestandteile den besonderen Eigenschaften und Erfordernissen der Haut von Männern, Frauen und Kindern anzupassen. So etwa werden bei stark behaarter Haut, wie sie meist Männer haben, durch eine stark shampoonierende Wirkung des Präparates die Verunreinigungen auch von den Haaren leicht abgelöst und beseitigt. Für die Haut von Frauen und Kindern, insbesondere von Säuglingen, lässt sich eine milde, wahlweise auch mit Glanzeffekten verbundene Wirkung erzielen.

Ein weiterer Vorteil der Erfindung ist darin gelegen, dass die Präparate ohne jede Änderung der Zusammensetzung wahlweise entweder in Form der fertigen Emulsion oder als Spray angewendet werden können. Eine besondere Ausführungsform liegt dabei in der unmittelbaren Beimischung oder Zuordnung von Treibgas oder Druckluft zur Erzielung eines versprühbaren Präparates, wobei im Falle der Druckluft die übliche Pumpflasche angewendet und damit der in jüngster Zeit unerwünschte Gebrauch von Treibgasen auf Fluorkohlenwasserstoffbasis vermieden werden kann. Eine weitere vorteilhafte Anwendungsmöglichkeit ist durch Aufbringen des erfindungsgemässen Präparates, insbesondere mit einem Mindestgehalt von 75 Gew.-% Wasser, zum Gebrauch als Feuchttüchlein gegeben.

Besonders gut brauchbare Präparate erhält man, wie bereits erwähnt, wenn die Mengenverhältnisse der einzelnen Komponenten zueinander bevorzugt in den folgenden Grenzen liegen:

Komponente 1    4 bis 16 Gew.-%
Komponente 2    4 bis 8 Gew.-%
Komponente 3    2 bis 8 Gew.-%
Komponente 4    4 bis 16 Gew.-%
Komponente 5    4 bis 10 Gew.-%
Komponente 6    5 bis 20 Gew.-%
Komponente 7    0,1 bis 2 Gew.-% (Aldehydbasis)
                 0,01 bis 0,1 Gew.-% (Chlorhexidinbasis)
Komponente 8    0,05 bis 1 Gew.-%
Komponente 9    Rest

Die einzelnen Komponenten 1, 2 und 3, die für das neue Hautpflege- und Hautreinigungsmittel charakteristisch sind, umfassen jeweils Gruppen von Verbindungen, die sich für den erfindungsgemässen Zweck besonders eignen. Zu diesen Komponentengruppen gehören beispielsweise:

1) Synthetische Wasch- bzw. Reinigungsrohstoffe:
   a) Alkyldiglykoläthersulfatnatriumsalz mit $C_{12}$-$C_{14}$-Alkyl
   b) Alkyltriglykoläthersulfatnatriumsalz mit $C_{12}$-$C_{14}$-Alkyl
   c) Alkylsulfattriäthanolammoniumsalz mit $C_{12}$-$C_{14}$-Alkyl
   d) neutralisiertes Fettsäuresarkosid auf Palmkern- und Kokosfettsäurebasis
   e) Fettsäuresarkosidnatriumsalz auf Laurinsäurebasis
   f) Fettsäuremethyltauridnatriumsalz auf Ölsäurebasis

2) Emulgatoren und/oder selbstemulgierende Grundlagen:
   a) Mono-, Di- und Tri(alkyltetraglykoläther)-o-phosphorsäureester (Alkyl = $C_{12}$-$C_{14}$)
   b) Mono-, Di- und Tri(alkyloctaglykoläther)-o-phosphorsäureester (Alkyl = $C_{18}$ ungesättigt)
   c) Mono-, Di- und Trialkyl-o-phosphorsäureester (Alkyl = $C_{18}$ ungesättigt)

d) Fettalkoholpolyglykoläther auf Oleylalkoholbasis
3) Perl- und/oder Seidenglanzgrundstoffe:
   a) Alkyläthersulfate zusammen mit Perlglanzbildnern und Schaumstabilisatoren
   b) Alkyläthersulfate zusammen mit Seidenglanzbildnern

Als Mineralölkomponente 4 wird vorteilhaft Paraffinöl der Bezeichnung *Paraffinum subliquidum* verwendet. Zusammen mit den übrigen Komponenten, wobei die Alkoholkomponente als erfrischender und zugleich die Reinigung unterstützender Anteil wirksam ist, lässt sich eine Vielzahl von Zusammensetzungen herstellen, wobei die für jede Gruppe beispielhaft angeführten Komponenten 1, 2, 4, 5, 6, 7 und 8 jeweils innerhalb dieser Gruppen untereinander austauschbar sind. Werden dabei die bereits erwähnten quantitativen Mengenverhältnisse gewahrt, so ergeben sich in jedem Falle gute Präparate, die den bekannten Mitteln überlegen sind.

Anhand der nachfolgenden Ausführungsbeispiele wird die Erfindung, ohne sie hierauf zu beschränken, weiter erläutert. Die einzelnen Komponenten sind dabei entweder durch die Zugehörigkeit zu der in Betracht kommenden Komponentengruppe 1 bis 9, ggf. durch die jeweilige Untergruppe a, b usw., oder durch den Namen der einzelnen Substanz bezeichnet. Die Zahlenwerte bedeuten immer Gewichtsprozente.

*Beispiel I:*

*Standardmittel für die Hautpflege und Hautreinigung im Bereich der Ausscheidungsorgane, auch bei aufgriebenen Hautpartien*

IA) Allgemeine Zusammensetzung:

| | |
|---|---|
| 2) selbstemulgierende Grundlagen | 4 bis 12 |
| 4) Mineralölkomponente | 3 bis 20 |
| 5) hygroskopischer Hautpflegeanteil | 3 bis 15 |
| 6) niedere Alkohole | 5 bis 20 |
| 7) Desinfektionsmittel, Aldehydbasis | 0,1 bis 2 |
| bzw. Chlorhexidinbasis | 0,01 bis 0,1 |
| 8) Duftöle | 0,05 bis 1 |
| 9) Wasser | 84,85 bis 30 |

Spezielle Zusammensetzungen:

| IB) | | IC) | |
|---|---|---|---|
| 2a) | 2 bis 6 | 2d) | 4 bis 12 |
| 4) | 3 bis 20 | 4) | 3 bis 20 |
| 5) | 3 bis 15 | 5) | 3 bis 15 |
| 6) Isopropanol | 5 bis 20 | 6) Isopropanol | 5 bis 20 |
| 7) Formaldehyd | 0,1 bis 2 | 7) Glyoxal | 0,1 bis 2 |
| 8) | 0,05 bis 1 | 8) | 0,05 bis 1 |
| 9) | 84,85 bis 30 | 9) | 84,85 bis 30 |

*Beispiel II:*

*Standardzusammensetzung eines Hautreinigungs- und Hautpflegemittels gemäss der Erfindung*

Allgemeine Zusammensetzung:

| | |
|---|---|
| 1) synthetische Wasch- bzw. Reinigungsrohstoffe | 1 bis 20 |
| 2) Emulgatoren bzw. selbstemulgierende Grundlagen | 3 bis 10 |
| 4) Mineralölkomponente | 2 bis 17 |
| 5) hygroskopischer Hautpflegeanteil | 2 bis 12 |
| 6) niedere Alkohole | 4 bis 16 |
| 7) Desinfektionsmittel, Aldehydbasis | 0,1 bis 2 |
| bzw. Chlorhexidinbasis | 0,01 bis 0,1 |
| 8) Duftöle | 0,05 bis 1 |
| 9) Wasser | 87,85 bis 22 |

*Beispiel III:*

*Spezielle Hautreinigungs- und -pflegemittel für Männer*

| IIIA) | | IIIB) | |
|---|---|---|---|
| 1c) | 1 bis 20 | 1d) | 1 bis 20 |
| 2b) | 1,5 bis 5 | 2a) | 1,5 bis 5 |
| 2d) | 1,5 bis 5 | 2d) | 1,5 bis 5 |
| 4) | 2 bis 17 | 4) | 2 bis 17 |
| 5) | 2 bis 12 | 5) | 2 bis 12 |
| 6) | 4 bis 16 | 6) | 4 bis 16 |
| 7) | 0,01 bis 2 | 7) | 0,01 bis 2 |
| 8) | 0,05 bis 1 | 8) | 0,05 bis 1 |
| 9) | Rest | 9) | Rest |

| IIIC) | | IIID) | |
|---|---|---|---|
| 1b) | 1 bis 7 | 1f) | 1 bis 20 |
| 1c) | 1 bis 7 | 2a) | 1,5 bis 5 |
| 1d) | 1 bis 7 | 2d) | 1,5 bis 5 |
| 2a) | 3 bis 10 | | |
| 4) | 2 bis 17 | 4) | 2 bis 17 |
| 5) | 2 bis 12 | 5) | 2 bis 12 |
| 6) | 4 bis 16 | 6) | 4 bis 16 |
| 7) | 0,01 bis 2 | 7) | 0,01 bis 2 |
| 8) | 0,05 bis 1 | 8) | 0,05 bis 1 |
| 9) | Rest | 9) | Rest |

*Beispiel IV:*

*Spezielle Hautreinigungs- und -pflegemittel für Frauen*

| IVA) | | IVB) | | IVC) | |
|---|---|---|---|---|---|
| 1a) | 1 bis 20 | 1b) | 1 bis 10 | 1b) | 1 bis 10 |
| 2b) | 1,5 bis 5 | 2c) | 1,5 bis 5 | 2a) | 1,5 bis 5 |
| 2d) | 1,5 bis 5 | 2d) | 1,5 bis 5 | 2d) | 1,5 bis 5 |
| | | 3a) | 1 bis 10 | 3b) | 1 bis 10 |
| 4) | 2 bis 17 | 4) | 2 bis 17 | 4) | 2 bis 17 |
| 5) | 2 bis 12 | 5) | 2 bis 12 | 5) | 2 bis 12 |
| 6) | 4 bis 16 | 6) | 4 bis 16 | 6) | 4 bis 16 |
| 7) | 0,01 bis 2 | 7) | 0,01 bis 2 | 7) | 0,01 bis 2 |
| 8) | 0,05 bis 1 | 8) | 0,05 bis 1 | 8) | 0,05 bis 1 |
| 9) | Rest | 9) | Rest | 9) | Rest |

*Beispiel V:*

*Spezielle Hautreinigungs- und -pflegmittel für Kinder, insbesondere Säuglinge*

| | |
|---|---|
| 1a) | 1 bis 17,5 |
| 2a) | 1,5 bis 5 |
| 2d) | 1,5 bis 5 |
| 4) | 2 bis 17 |
| 5) | 2 bis 12 |
| 6) | 2 bis 10 |
| 7) | 0,1 bis 1,5 bzw. 0,01 bis 0,1 |
| 8) | 0,05 bis 1 |
| 9) | Rest |

## Patentansprüche

1. Mittel zur Reinigung und Pflege der Haut des Menschen im Bereich der Ausscheidungsorgane und der angrenzenden Körperteile, mit einem Gehalt an desinfizierend wirkenden, ggf. noch zusätzlich desodorisierend, erfrischend und/oder parfümierend wirkenden, hautverträglichen Komponenten, gekennzeichnet durch die folgende Zusammensetzung der ggf. versprühbaren Emulsion:

1) synthetische Wasch- und Reinigungsrohstoffe auf Basis von Alkyläthersulfaten bzw. Alkylsulfaten und/oder Fettsäuresarkosiden bzw. Fettsäuremethyltauriden
  1 bis 21, vorzugsweise 4 bis 16 Gew.-%
2) Emulgatoren bzw. selbstemulgierende Grundlagen auf Basis von Alkylpolyglykolätherphosphaten und/oder Fettalkoholpolyglykoläthern, wobei ggf. die selbstemulgierenden Grundlagen an die Stelle der Komponente (1) treten können
  1 bis 12, vorzugsweise 4 bis 8 Gew.-%
3) ggf. Perl- und/oder Seidenglanzgrundstoffe auf Basis von Alkyläthersulfaten
  1 bis 10, vorzugsweise 2 bis 8 Gew.-%
4) viskose hautverträgliche Mineralöle, insbesondere Paraffinöl
  2 bis 20, vorzugsweise 4 bis 16 Gew.-%
5) Glycerin und/oder Glykole
  2 bis 15, vorzugsweise 4 bis 10 Gew.-%
6) niedere aliphatische Alkohole
  2 bis 20, vorzugsweise 5 bis 20 Gew.-%
7) Desinfektionsmittelkomponente auf Aldehydbasis  0,1 bis 2 Gew.-%
oder auf Chlorhexidinbasis
  0,01 bis 0,1 Gew.-%
8) Duftstoffkomponente  0,05 bis 1 Gew.-%
9) Wasser  Rest

2. Mittel nach Anspruch 1, insbesondere zum Schutz der Haut im Bereich der Ausscheidungsorgane vor Rötungen und Entzündungen, gekennzeichnet durch die folgende Zusammensetzung:

2) selbstemulgierende Grundlagen, insbesondere Fettalkoholpolyglykoläther auf Oleylalkoholbasis, ggf. zusammen mit Alkyltetra- oder -octaglykolätherphosphaten oder Alkylphosphaten  4 bis 12 Gew.-%
4) Paraffinöl  3 bis 20 Gew.-%
5) Glycerin und/oder Glykole  3 bis 15 Gew.-%
6) niedere aliphatische Alkohole, insbesondere Isopropanol  5 bis 20 Gew.-%
7) Formaldehyd oder Glyoxal  0,1 bis 2 Gew.-%
bzw. Chlorhexidin  0,01 bis 0,1 Gew.-%
8) Duftstoffkomponente  0,05 bis 1 Gew.-%
9) Wasser  Rest

3. Mittel nach Anspruch 1, insbesondere zur Hautreinigung und -pflege für Männer, gekennzeichnet durch die folgende Zusammensetzung:

1) synthetische Wasch- und Reinigungsrohstoffe, insbesondere Alkyltriglykoläthersulfatnatriumsalz, Alkylsulfattriäthanolammoniumsalz, neutralisiertes Fettsäuresarkosid und Fettsäuremethyltauridnatriumsalz  1 bis 21 Gew.-%
2) Emulgatoren bzw. selbstemulgierende Grundlagen, insbesondere Mono- Di-, und Tri(al-kyltetraglykoläther)-o-phosphorsäureester, Mono-, Di- und Tri(alkyloctaglykoläther)-o-phosphorsäureester, Fettalkoholpolyglykoläther auf Oleylalkoholbasis 1,5 bis 10 Gew.-%
4) Paraffinöl  2 bis 17 Gew.-%
5) Glycerin und/oder Glykole  2 bis 12 Gew.-%
6) niedere aliphatische Alkohole, insbesondere Isopropanol  4 bis 16 Gew.-%
7) Formaldehyd oder Glyoxal  0,1 bis 2 Gew.-%
bzw. Chlorhexidin  0,01 bis 0,1 Gew.-%
8) Duftstoffkomponente  0,05 bis 1 Gew.-%
9) Wasser  Rest

4. Mittel nach Anspruch 1, insbesondere zur Hautreinigung und -pflege für Frauen, gekennzeichnet durch die folgende Zusammensetzung:

1) synthetische Wasch- und Reinigungsrohstoffe, insbesondere Alkyldiglykoläthersulfatnatriumsalz und Alkyltriglykoläthersulfatnatriumsalz  1 bis 20 Gew.-%
2) Emulgatoren bzw. selbstemulgierende Grundlagen, insbesondere Mono, Di- und Tri(al-kyltetraglykoläther)-o-phosphorsäureester, Mono-, Di- und Tri(alkyloctaglykoläther)-o-phosphorsäureester, Mono-, Di- und Trialkyl-o-phosphorsäureester, Fettalkoholpolyglykoläther auf Oleylalkoholbasis 1,5 bis 10 Gew.-%
3) Alkyläthersulfate zusammen mit Perlglanzbildnern und Schaumstabilisatoren bzw. Seidenglanzbildnern  1 bis 10 Gew.-%
4) Paraffinöl  2 bis 17 Gew.-%
5) Glycerin und/oder Glykole  2 bis 12 Gew.-%
6) niedere aliphatische Alkohole, insbesondere Isopropanol  4 bis 16 Gew.-%
7) Formaldehyd oder Glyoxal  0,1 bis 2 Gew.-%
bzw. Chlorhexidin  0,01 bis 0,1 Gew.-%
8) Duftstoffkomponente  0,05 bis 1 Gew.-%
9) Wasser  Rest

5. Mittel nach Anspruch 1, insbesondere zur Hautreinigung und -pflege für Kinder und Säuglinge, gekennzeichnet durch die folgende Zusammensetzung:

1) synthetische Wasch- und Reinigungsrohstoffe, insbesondere Alkyldiglykoläthersulfatnatriumsalz  1 bis 17,5 Gew.-%
2) Emulgatoren und selbstemulgierende Grundlagen, insbesondere Mono-, Di- und Tri(alkyltetraglykoläther)-o-phosphorsäureester und Fettalkoholpolyglykoläther auf Oleylalkoholbasis  1,5 bis 10 Gew.-%
4) Paraffinöl  2 bis 17 Gew.-%
5) Glycerin und/oder Glykole  2 bis 12 Gew.-%
6) niedere aliphatische Alkohole, insbesondere Isopropanol  2 bis 10 Gew.-%
7) Formaldehyd oder Glyoxal 0,1 bis 1,5 Gew.-%
bzw. Chlorhexidin  0,01 bis 0,1 Gew.-%
8) Duftstoffkomponente  0,05 bis 1 Gew.-%
9) Wasser  Rest

## Claims

1. Preparation for the cleaning and care of the human skin in the excretory organs region and of the body adjacent to said organs, comprising components compatible with the skin exercising a disinfectant and optionally also deodorizing, refreshing and/or perfuming effect, characterized by the following composition of an optionally sprayable emulsion:

1) synthetic washing and cleansing raw materials on the basis of alkyl ether sulfates and alkyl sulfates, respectively, and/or fatty acid sarcosides and fatty acid methyl taurides, respectively 1 to 21, preferably 4 to 16% by weight

2) emulsifiers and self-emulsifying materials, respectively, on the basis of alkyl polyglycol ether phosphates and/or fatty alcohol polyglycol ethers, said self-emulsifying materials optionally replacing component (1) 1 to 12, preferably 4 to 8% by weight

3) optionally pearl- and/or silk-gloss imparting raw materials on the basis of alkyl ether sulfates 1 to 10, preferably 2 to 8% by weight

4) viscous mineral oils compatible with the skin, especially paraffin oil 2 to 20, preferably 4 to 16% by weight

5) glycerol and/or glycols 2 to 15, preferably 4 to 10% by weight

6) lower aliphatic alcohols 2 to 20, preferably 5 to 20% by weight

7) disinfectant component on an aldehyde basis 0.1 to 2% by weight or on chlorohexidine basis 0.01 to 0.1% by weight

8) perfuming component 0.05 to 1% by weight
9) water     remainder

2. Preparation as claimed in Claim 1, particularly for protecting the skin in the area of the excretory organs against reddening and inflammations, characterized by the following composition:

2) self-emulsifying materials, especially fatty alcohol polyglycol ether based on oleyl alcohol, optionally in combination with alkyl tetra- or octaglycol ether phosphates or alkyl phosphates     4 to 12% by weight
4) paraffin oil     3 to 20% by weight
5) glycerol and/or glycols   3 to 15% by weight
6) lower aliphatic alcohols, especially isopropanol     5 to 20% by weight
7) formaldehyde or glyoxal 0.1 to 2% by weight
or chlorohexidine, respectively 0.01 to 0.1% by weight

8) perfuming component 0.05 to 1% by weight
9) water     remainder

3. Preparation as claimed in Claim 1, particularly for cleansing and caring for the skin of men, characterized by the following composition:

1) synthetic washing and cleansing raw materials, especially alkyl triglycol ether sulfate sodium salt, alkyl sulfate triethanol ammonium salt, neutralized fatty acid sarcoside and fatty acid methyltauride sodium salt 1 to 21% by weight

2) emulsifiers and self-emulsifying materials, respectively, especially mono-, di- and tri(alkyltetraglycolether)orthophosphoric acid esters, mono-, di- and tri(alkyloctaglycolether)orthophosphoric acid esters, fatty alcohol polyglycol ether based on oleyl alcohol 1.5 to 10% by weight

4) paraffin oil     2 to 17% by weight
5) glycerol and/or glycols   2 to 12% by weight
6) lower aliphatic alcohols, especially isopropanol     4 to 16% by weight
7) formaldehyde or glyoxal 0.1 to 2% by weight
or chlorohexidine, respectively 0.01 to 0.1% by weight

8) perfuming component 0.05 to 1% by weight
9) water     remainder

4. Preparation as claimed in claim 1, particularly for cleansing and caring for the skin of women, characterized by the following composition:

1) synthetic washing and cleansing raw materials, especially alkyl diglycol ether sulfate sodium salt and alkyl triglycol ether sulfate sodium salt     1 to 20% by weight

2) emulsifiers and self-emulsifying materials, respectively, especially mono-, di- and tri(alkyltetraglycolether)orthophosphoric acid esters, mono-, di- and tri(alkyloctaglycolether)orthophosphoric acid esters, mono-, di- and trialkylorthophosphoric acid esters, fatty alcohol polyglycol ether based on oleyl alcohol 1.5 to 10% by weight

3) alkyl ether sulfates in combination with pearl-gloss imparting agents and foam stabilizers or silk-gloss imparting agents, respectively 1 to 10% by weight

4) paraffin oil     2 to 17% by weight
5) glycerol and/or glycols 2 to 12% by weight
6) lower aliphatic alcohols, especially isopropanol     4 to 16% by weight
7) formaldehyde or glyoxal 0.1 to 2% by weight
or chlorohexidine, respectively 0.01 to 0.1% by weight

8) perfuming component 0.05 to 1% by weight
9) water     remainder

5. Preparation as claimed in Claim 1, particularly for cleansing and caring for the skin of children and babies, characterized by the following composition:

1) synthetic washing and cleansing raw materials, especially alkyl diglycol ether sulfate sodium salt     1 to 17.5% by weight

2) emulsifiers and self-emulsifying materials, especially mono-, di- and tri(alkyltetraglycolether)orthophosphoric acid esters and fatty alcohol polyglycol ether based on oleyl alcohol 1.5 to 10% by weight

4) paraffin oil     2 to 17% by weight
5) glycerol and/or glycols 2 to 12% by weight
6) lower aliphatic alcohols, especially isopropanol     2 to 10% by weight
7) formaldehyde or glyoxal 0.1 to 1.5% by weight or chlorohexidine, respectively 0.01 to 0.1% by weight

8) perfuming component 0.05 to 1% by weight
9) water     remainder

## Revendications

1. Préparation pour le nettoyage et les soins de la peau humaine dans la région des organes d'excrétion et des parties avoisinantes du corps, contenant des composants tolérés par la peau, exerçant un effet désinfectant et éventuellement, en outre, un effet désodorisant, rafraîchissant et/ou parfumant, caractérisée par la constitution suivante de l'émulsion éventuellement atomisable:

1) produits synthétiques de lavage et de nettoyage à base d'alcoyléthersulfates ou alcoylsulfates et/ou de sarcosides d'acides gras ou méthyltaurides d'acides gras
1 à 21, de préférence 4 à 16% en poids

2) émulsionnants ou bases auto-émulsionnantes à base d'alcoylpolyglycolétherphosphates et/ou éthers polyglycoliques d'alcools gras, les bases auto-émulsionnantes pouvant éventuellement remplacer le composant (1)
1 à 12, de préférence 4 à 8% en poids

3) éventuellement matières premières nacrées et/ou chatoyantes à base d'alcoyléthersulfates
1 à 10, de préférence 2 à 8% en poids

4) huiles minérales visqueuses tolérées par la peau, en particulier huile de paraffine
2 à 20, de préférence 4 à 16% en poids

5) glycérine et/ou glycols
2 à 15, de préférence 4 à 10% en poids

6) alcools aliphatiques inférieurs
2 à 20, de préférence 5 à 20% en poids

7) composant désinfectant à base d'aldéhyde
0,1 à 2% en poids
ou à base de chlorhexidine
0,01 à 0,1% en poids

8) composant parfumant      0,05 à 1% en poids

9) eau      reste

2. Préparation suivant la revendication 1, en particulier pour la protection de la peau dans la région des organes d'excrétion contre les rougeurs et inflammations, caractérisée par la constitution suivante:

2) bases auto-émulsionnantes, en particulier éther polyglycolique d'alcool gras à base d'alcool oléylique, éventuellement avec des alcoyltétra- ou octaglycolétherphosphates ou alcoylphosphates      4 à 12% en poids

4) huile de paraffine      3 à 20% en poids

5) glycérine et/ou glycols      3 à 15% en poids

6) alcools aliphatiques inférieurs, en particulier isopropanol      5 à 20% en poids

7) formaldéhyde ou glyoxal      0,1 à 2% en poids
ou chlorhexidine      0,01 à 0,1% en poids

8) composant parfumant      0,05 à 1% en poids

9) eau      reste

3. Préparation suivant la revendication 1, en particulier pour le nettoyage et les soins de la peau des hommes, caractérisée par la constitution suivante:

1) produits synthétiques de lavage et de nettoyage, en particulier sel de sodium d'alcoyltriglycoléthersulfate, sel de triéthanolammonium d'alcoylsulfate, sarcoside d'acide gras neutralisé et sel de sodium de méthyltauride d'acide gras      1 à 21% en poids

2) émulsionnants ou bases auto-émulsionnantes, en particulier ester orthophosphorique de mono-, di- et tri(alcoyltétraglycoléther), ester orthophosphorique de mono-, di et tri(alcoyloctaglycoléther), éther polyglycolique d'alcool gras à base d'alcool oléylique
1,5 à 10% en poids

4) huile de paraffine      2 à 17% en poids

5) glycérine et/ou glycols      2 à 12% en poids

6) alcools aliphatiques inférieurs, en particulier isopropanol      4 à 16% en poids

7) formaldéhyde ou glyoxal      0,1 à 2% en poids
ou chlorhexidine      0,01 à 0,1% en poids

8) composant parfumant      0,05 à 1% en poids

9) eau      reste

4. Préparation suivant la revendication 1, en particulier pour le nettoyage et les soins de la peau des femmes, caractérisée par la constitution suivante:

1) produits synthétiques de lavage et de nettoyage, en particulier sel de sodium d'alcoyldiglycoléthersulfate et sel de sodium d'alcoyltriglycoléthersulfate      1 à 20% en poids

2) émulsionnants ou bases auto-émulsionnantes, en particulier ester orthophosphorique de mono-, di- et tri(alcoyltétraglycoléther), ester orthophosphorique de mono-, di- et tri(alcoyloctaglycoléther), orthophosphate de mono-, di- et trialcoyle, éther polyglycolique d'alcool gras à base d'alcool oléylique
1,5 à 10% en poids

3) alcoyléthersulfates avec des agents nacrants et stabilisants de la mousse ou agents chatoyants
1 à 10% en poids

4) huile de paraffine      2 à 17% en poids

5) glycérine et/ou glycols      2 à 12% en poids

6) alcools aliphatiques inférieurs, en particulier isopropanol      4 à 16% en poids

7) formaldéhyde ou glyoxal      0,1 à 2% en poids
ou chlorhexidine      0,01 à 0,1% en poids

8) composant parfumant      0,05 à 1% en poids

9) eau      reste

5. Préparation suivant la revendication 1, en particulier pour le nettoyage et les soins de la peau des enfants et des nourrissons, caractérisée par la constitution suivante:

1) produits synthétiques de lavage et de nettoyage, en particulier sel de sodium d'alcoyldiglycoléthersulfate      1 à 17,5% en poids

2) émulsionnants et bases auto-émulsionnantes, en particulier ester orthophosphorique de mono-, di- et tri(alcoyltétraglycoléther) et éther polyglycolique d'alcool gras à base d'alcool oléylique      1,5 à 10% en poids

4) huile de paraffine      2 à 17% en poids

5) glycérine et/ou glycols      2 à 12% en poids

6) alcools aliphatiques inférieurs, en particulier isopropanol      2 à 10% en poids

7) formaldéhyde ou glyoxal  0,1 à 1,5% en poids
ou chlorhexidine      0,01 à 0,1% en poids

8) composant parfumant      0,05 à 1% en poids

9) eau      reste